# EUROPEAN PATENT SPECIFICATION

(11) **EP 3 317 790 B1**
(45) Date of publication and mention of the grant of the patent: **07.02.2024**
(21) Application number: 16747929.4
(22) Date of filing: 30.06.2016
(51) Int. Cl.: G16H 50/20, G16H 50/30, G16H 50/50, G16H 40/63

(54) **MULTIPLE SENSORS FOR BIOMETRIC ANALYSIS**
MULTIPLE SENSOREN FÜR BIOMETRISCHE ANALYSE
CAPTEURS MULTIPLES POUR L'ANALYSE BIOMÉTRIQUE

(30) Priority: 01.07.2015 US 201562187712 P
(43) Date of publication of application: 09.05.2018
(73) Proprietor: Verily Life Sciences LLC, Mountain View, CA 94043 (US)
(72) Inventor: WASSON, Jaclyn, Leverett, Mountain View, CA 94043 (US); BIEDERMAN, William, James, Mountain View, CA 94043 (US); OTIS,Brian, Mountain View, CA 94043 (US); LIU, Zenghe, Mountain View, CA 94043 (US)
(74) Representative: Mewburn Ellis LLP
(86) International application number: PCT/US2016/040227
(87) International publication number: WO 2017/004284

(56) References cited:
- WO-A1-2011/039745
- US-A1- 2011 015 510
- Anonymous: "Sensor fusion - Wikipedia, the free encyclopedia", , 22 June 2015 (2015-06-22), XP055301366, Retrieved from the Internet: URL:https://en.wikipedia.org/w/index.php?t itle=Sensor_fusion&oldid=668209547 [retrieved on 2016-09-09]
- Anonymous: "Electroencephalography - Wikipedia", , 21 June 2015 (2015-06-21), XP055511304, Retrieved from the Internet: URL:https://en.wikipedia.org/w/index.php?t itle=Electroencephalography&oldid=66794563 7 [retrieved on 2018-10-01]

## Description

### BACKGROUND

Unless otherwise indicated herein, the materials described in this section are not prior art to the claims in this application and are not admitted to be prior art by inclusion in this section.

Biometric sensors may measure one or more physiological indicators of an individual. For example, a transdermal sensor may be configured to provide continuous or periodic information indicative of a concentration of an analyte, such as glucose.

However, application of some biometric sensors may be painful and/or time-consuming. Furthermore, if the sensor is not applied to a proper location on the body, an individual may replace the sensor with another sensor until the proper location is found. Also, biometric sensors may have an internal offset or error unique to each sensor. Additionally, such sensors may not always report accurate and/or comparable measurements.

US2011/015510A1 discloses an analyte monitor including a subcutaneously implantable sensor for detecting an analyte, a sensor control unit, and a display unit. The display unit is configured to receive sensor data transmitted from the sensor control unit and display an indication of a level of the analyte. The analyte monitor may also be part of a drug delivery system for altering the level of the analyte based on data obtained using the sensor.

"Sensor fusion - Wikipedia, the free encyclopedia" (https://en.wikipedia.org/w/index.php?title=Sensor_fusion&oldid=668209547) discloses sensor fusion, the combining of sensory data or data derived from sensory data from disparate sources such that the resulting information has less uncertainty than would be possible when these sources were used individually.

WO2011/039745A4 discloses a continuous non-interfering health monitoring and alert system designed for use by a health living being but also suitable for a non-healthy living being. The system includes a control module, a communication unit and one or more sensors. The sensors can be in-vivo nano-sensors, micro-sensors, subcutaneous, wearable or implanted sensors. The control unit includes an analysis subsystem, having a processing unit and an alerting unit. Each of the sensors is configured to detect a predetermined physiological or chemical parameter of the living being. The communication unit is facilitated to transmit the detected parameters to the analysis subsystem. The processor analyzes the detected parameters to thereby determine if the health state of the monitored living being is abnormal. When at least one detected parameter or the health state is determined to be abnormal, the alerting unit is operatively activated to alert a predetermined alert receiving entity.

"Electroencephalography - Wikipedia, the free encyclopedia" (https://en.wikipedia.org/w/index.php?title=Electroencephalography&oldid=667945637) discloses electroencephalography (EEG), a typically non-invasive method to record electrical activity of the brain along the scalp. EEG measures voltage fluctuations resulting from ionic current within the neurons of the brain. In clinical contexts, EEF refers to the recording of the brain's spontaneous electrical activity over a period of time, as recorded from multiple electrodes placed on the scalp. Diagnostic applications generally focus on the spectral content of EEF, that is, the type of neural oscillations that can be observed in EEG signals.

### SUMMARY

The scope of the invention is defined by the appended claims. Other aspects, embodiments, and implementations will become apparent to those of ordinary skill in the art by reading the following detailed description, with reference where appropriate to the accompanying drawings.

### BRIEF DESCRIPTION OF THE FIGURES

Figure 1 is a block diagram of a system according to an example embodiment.
Figure 2A illustrates sensors and respective mounting locations on a living body according to an example embodiment.
Figure 2B illustrates graphs of sensor data according to example embodiments.
Figure 3A illustrates a mobile device according to an example embodiment.
Figure 3B illustrates a mobile device according to an example embodiment.
Figure 4A illustrates a body-mountable device according to an example embodiment.
Figure 4B illustrates a cross-sectional view of a body-mountable device according to an example embodiment.
Figure 5 illustrates a system according to an example embodiment.
Figure 6 illustrates a method according to an example embodiment.
Figure 7 illustrates a method according to an example embodiment.

### DETAILED DESCRIPTION

In the following detailed description, reference is made to the accompanying figures, which form a part hereof. In the figures, similar symbols typically identify similar components, unless context dictates otherwise. The illustrative embodiments described in the detailed description, figures, and claims are not meant to be limiting.

Further, while embodiments disclosed herein make reference to use on or in conjunction with a living human body, it is contemplated that the disclosed methods, systems and devices may be used in any environment where measuring a physiological parameter with a plurality of sensors is desirable. The environment may be any living or non-living body or a portion thereof, etc. For example, one of skill in the art will recognize that the embodiments disclosed herein may be used to sense many different physiological parameters. Moreover, while the present disclosure describes embodiments for use *in vivo,* one of skill in the art will also recognize that *in vitro* applications are possible as well.

The terms "body-mountable device" or "wearable device," as used in this disclosure, refer to any device that is capable of being worn at, on or in proximity to a body surface, such as a wrist, ankle, waist, chest, or other body part.

### Overview

In an example embodiment, a system may include multiple sensors positioned at one or more locations on a living body. For instance, two transdermal glucose sensors may be placed on two different locations on the living body. The multiple sensors may be placed in various locations on the body, e.g. forearm, bicep, tricep, shoulder, chest, abdomen, buttocks, leg, back, etc. In some example embodiments, at least two of the multiple sensors may be located within the same transdermal patch.

Alternatively or additionally, at least one sensor of the multiple sensors may include a sensor implanted in the living body. For example, an individual may have a temporary or permanent sensor implant configured to provide temperature data.

The system may include a controller configured to receive sensor data from each of the multiple sensors. The controller may include a memory and a processor. In an example embodiment, the controller may include a mobile device, such as a smartphone, smartwatch, wearable computing device, or another type of computer. The controller and the multiple sensors may communicate via a near-field communication (NFC), WiFi, or a Bluetooth Low Energy (BLE) wireless link. In an example embodiment, the controller may receive information indicative of a concentration of glucose from each of the transdermal glucose sensors.

Each of the multiple sensors may provide sensor data to the controller on a continuous and/or as-requested basis. For example, in the scenario in which the multiple sensors are configured to communicate with the controller using an NFC link, when the controller is in physical proximity to a biometric sensor, that sensor may upload stored sensor data to the controller. In an example embodiment, a mobile device may come into physical proximity with a glucose sensor incorporated into a transdermal patch. In response to the mobile device being in proximity to the sensor, the sensor may upload sensor data to the controller. The sensor data uploaded may include, for example, data stored since the last data upload to the controller or another set of sensor data.

In an alternative embodiment, where the sensors are operable to communicate with the controller via BLE or WiFi, the multiple sensors may provide sensor data to the controller on a periodic and/or continual basis. Alternatively, the multiple sensors may provide sensor data upon receipt of a request from the controller.

The controller correlates the sensor data from the multiple sensors to provide correlated data. As an example, the controller may provide correlated data based on an average of the sensor data, e.g. to determine a mean concentration of glucose. The controller provides correlated data based on a time-weighted average of the data based on the respective sensor age, e.g. sensor data from older sensors is discounted to a greater degree than sensor data from newer sensors. Yet further, the controller may be configured to determine an aging or unreliable sensor and discount or disregard the sensor data from that sensor when determining correlated data. For example, a biometric sensor may become bio-fouled over time and/or run out of power, e.g. due to battery life. In an example embodiment, the controller may determine correlated data based on time-weighted information indicative of an individual's glucose concentration.

While some examples herein relate to sensor data relating to a glucose concentration, it will be understood that other types of data and/or other physiological parameters are possible. For example, the sensor data may provide information indicative of a concentration of glycated hemoglobin (HbA_{1c}).

In an example embodiment, the controller may determine correlated data based on sensor data that is within a predetermined number of standard deviations from an average. In other words, outlying data points may be dismissed or discounted. Alternatively or additionally, the controller may determine correlated data based on historical data and/or historical deviations from an average. Yet further, the controller may determine correlated data based on environmental factors, such as temperature, humidity, etc.

In some embodiments, the controller may cause one or more of the multiple sensors to turn off in response to determining that the sensors have become bio-fouled, are providing unreliable data, or are older than a predetermined age threshold. In such a scenario, the system may conserve power and/or battery life by powering down unreliable sensors.

In scenarios where at least one sensor includes a temperature sensor, the controller may be configured to provide correlated data based on a temperature offset and/or a temperature-dependent accuracy of the biometric sensors.

In scenarios where the multiple sensors are positioned at different physical locations on the living body, the controller may be configured to provide correlated data based on the respective physical locations of each of the multiple sensors. For example, the multiple sensors may provide information regarding local or global metabolism rates of an analyte based on the respective location of the sensors. Alternatively or additionally, the time-response of sensors at may indicate how fast a concentration of an analyte may change based on their respective locations on the body, e.g. in response to the introduction of insulin.

The controller may determine a health state based on the correlated data being within a predetermined range. For example, the controller may determine a high glucose health state (e.g. a hyperglycemic state) based on the correlated data indicating a glucose concentration higher than 180 mg/dL.

In some example embodiments, the controller may determine a predicted health state based on the correlated data being within a predetermined range or following a predetermined trend. For example, the correlated data may be indicative of an increasing variability in the glucose concentration of an individual. In such a scenario, a predicted health state may include pre-diabetes. Other predicted health states may be possible.

The controller provides an indication based on the health state and , optionally, the predicted health state. In the above scenario, the controller may cause a display to provide a high glucose level alert indication. Other types of indications are possible, for example, the controller may cause a display to provide a historical graph or another type of aggregated data regarding the health state. In some example embodiments, the controller may be configured to provide various healthcare directives to a user. For instance, the indication may include a notification via a smartphone such as, "High Glucose Concentration, please administer insulin."

In the example in which the controller may determine a predicted health state, the indication may include a notification to make an appointment with a medical doctor or to carry out other actions, e.g. exercise, eat less sugary foods, etc. Other indications based on predicted health states are possible.

Additionally or alternatively, the controller may be operable to cause a transdermal patch to deploy a substance via one or more microneedles. That is, determination of a particular health state may trigger a deployment of a beneficial medicine so as to alleviate the health state. Other actions by the controller are possible in response to determining the health state.

In some embodiments, the controller may be communicatively coupled to a cloud computing network. In such a scenario, the controller and/or another computer system may be configured to aggregate anonymized health information from a plurality of individuals. As such, the controller and/or the other computer system may be configured to provide analytical and/or statistical data about particular types of sensor data. In an example embodiment, data may be aggregated so as to help healthcare providers and patients to manage various health conditions, e.g. diabetes or other diseases/symptoms.

By obtaining correlated data from multiple sensors, the systems and methods herein may improve the reliability of biometric sensor data while reducing sensor calibration operations and/or repeated applications of transdermal patches.

### System Examples

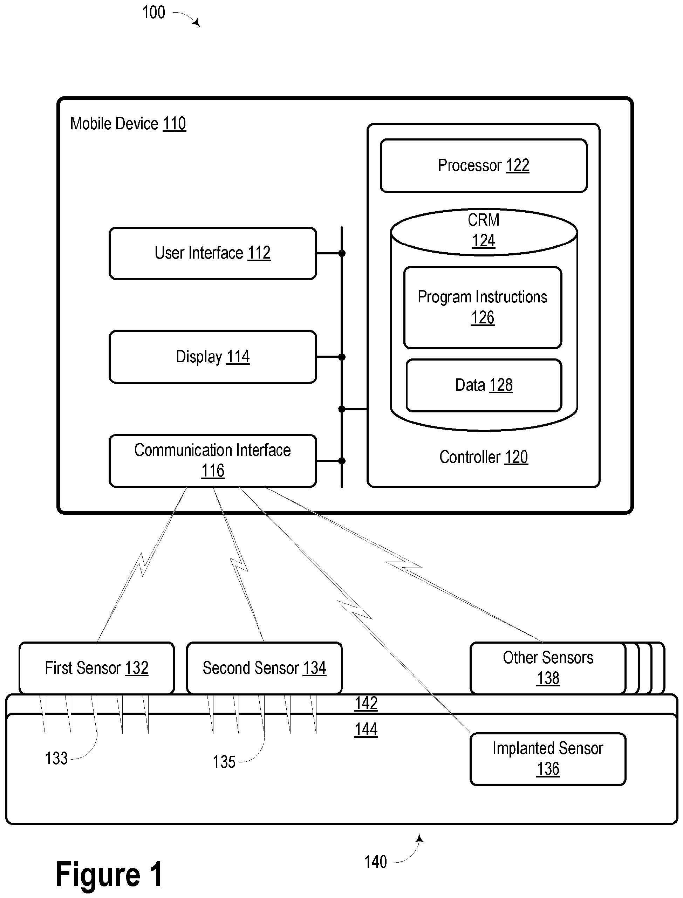

Figure 1 is a block diagram of a system 100 according to an example embodiment. System 100 includes a mobile device 110 and a plurality of sensors, which include a first sensor 132, a second sensor 134, an optional implanted sensor 136, and optional other sensors 138. The plurality of sensors may be in proximity to a living body 140. For example, the plurality of sensors may be physically coupled to an epidermal skin surface 142 of the living body 140. Some or all of the plurality of sensors may include transdermal sensors such as first sensor 132 and second sensor 134. Such transdermal sensors may include one or more respective microneedle arrays 133 and 135 configured to contact a dermal skin layer 144. Additionally or alternatively, the plurality of sensors may include one or more implanted sensors 136 implanted within the living body 140 and/or other sensors 138 that may be optionally coupled to an external surface of the living body 140.

In an example embodiment, at least two sensors may be collocated in a transdermal sensor patch. For example, a transdermal patch may include a 5x5 array of transdermal microneedle sensors, which may represent several sensors of the plurality of sensors. Additionally or alternatively, other sensors (e.g. a temperature sensor) may be included in the transdermal patch.

In a further embodiment, several transdermal patches, each of which may include a plurality of sensors, may be attached in various locations on the body. Other combinations of sensors and bodily attachment locations are possible

The plurality of sensors may include one or more types of sensors, which may include, but are not limited to transdermal sensors, adhesive sensors, body-mountable sensors, wearable sensors, clip-on sensors, implantable sensors, or other sensors configured to provide information indicative of a health state of a living body. The sensors of the plurality of sensors may be configured to measure one or more physiological parameters of the living body. For example, the sensors may be configured to measure one or more of: a concentration of glucose, a heart rate, a blood pressure, or a temperature.

The mobile device 110 may include a user interface 112, a display 114, a communication interface 116, and a controller 120. The communication interface 116 may be operable to provide a one or more communication links. The communication links may include one or more of a near field communication (NFC) link, a BLUETOOTH Low Energy (BLE) link, an ultra high frequency (UHF) radio frequency identification (RFID) link, or a WiFi link.

For example, at least some sensors from the plurality of sensors may be configured as a passive RFID tag, so as to communicate with the mobile device 110 via a UHF (e.g. 860-960 MHz) RFID link. In such a scenario, the mobile device 110 may act as a reader device for the sensors. That is, the mobile device 110 may transmit an interrogating electromagnetic field. In response to the interrogating electromagnetic field, the sensor(s) may transmit stored sensor data to the mobile device 110. Active RFID tags are also contemplated in the scope of the present disclosure. Other types of communication links are possible.

The controller 120 includes a processor 122, and a non-transitory computer-readable medium 124 (e.g., memory) that may store program instructions 126 and data 128. The program instructions 126 are executable by the processor 122 to cause the controller 120 to perform operations, as discussed below.

The program instructions 126 includes instructions for receiving, from the first sensor 132 of the plurality of sensors, first sensor data indicative of the physiological parameter. That is, the controller 120 may receive the first sensor data, which may relate to a concentration of an analyte, such as glucose. The program instructions 126 include instructions for receiving, from the second sensor 134 of the plurality of sensors, second sensor data indicative of the physiological parameter. In other words, the controller 120 may receive the second sensor data, which may also relate to the concentration of the same analyte (e.g. glucose).

The program instructions 126 includes instructions for correlating the first sensor data and the second sensor data to provide correlated data. The first and second sensor data are weighted based on an operational age of the respective sensor. For instance, the first and second sensor data may be averaged. Additionally or alternatively, other operations may be carried out so as to correlate the first and second sensor data. For example, the first and second sensor data may be weighted according a predicted or determined reliability. That is, sensor data received from the plurality of sensors may be weighted, discounted, and/or dismissed based on one or more factors such as an outlying data set/point, historical data trends/ranges, calibration data, and/or an operational state of the sensor.

The program instructions 126 include instructions for determining a health state based at least on the correlated data. The health state may include any sort of characterization of the health of a living body. For example, the health state may include a "normal" state, a hyperglycemic/hypoglycemic state, a fever, an elevated stress state, and/or a high blood pressure state. Many other health states are possible.

The program instructions 126 include instructions for providing an indication based on the determined health state. That is, the controller 120 may cause the user interface 112 and/or the display 114 to display an indication related to the determined health state.

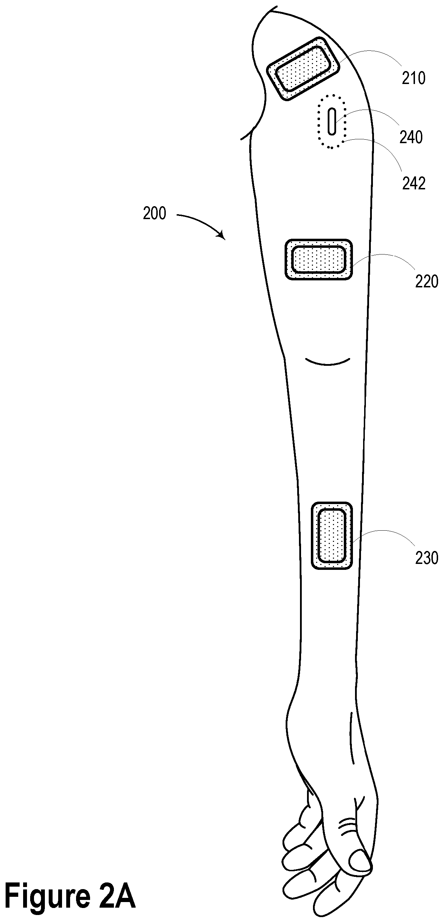

Figure 2A illustrates sensors 210, 220, 230, and 240 and respective mounting locations on a living body 200 according to an example embodiment. As illustrated, the living body 200 may include a human body, however living bodies of other species are contemplated herein. In an example embodiment, sensors 210, 220, and 230 may include transdermal sensors configured to sense a concentration of an analyte, such as glucose, in the living body 200. The sensors 210, 220, and 230 may be removably coupled to the living body 200 near the shoulder, bicep, and forearm, respectively. Sensor 240 may be an implanted sensor implanted beneath a skin portion 242 on a shoulder of the living body 200. Sensor 240 may be configured to sense a body temperature.

In some example embodiments, the sensors described herein need not be of the same type. For instance, the plurality of sensors may include a variety of sensors configured to sense various physiological parameters of a living body. Furthermore, sensor data received from a first sensor may help to calibrate and/or adjust sensor data received from a second sensor, or vice-versa. For example, a first sensor may include an implanted temperature sensor, e.g. sensor 240, and a second sensor may be one or more of sensors 210, 220, or 230. In such a scenario, the second sensor may exhibit a temperature-dependent sensor offset and/or temperature-dependent sensor performance. As such, temperature data received from the first sensor (sensor 240) may be used to adjust and/or calibrate the data (e.g. glucose concentration data) received from the second sensor (sensor 210). In other words, the temperature-dependent performance of the second sensor (or other sensors providing similar data) may be corrected, calibrated, or adjusted based on a measured body temperature. Other types of calibration and/or adjustment are possible. For instance, sensor data may be adjusted based on humidity, a sensor location, sensor movement, or other factors. In such ways, the data indicative of a physiological parameter may be adjusted so as to be more reliable, accurate, and/or reproducible.

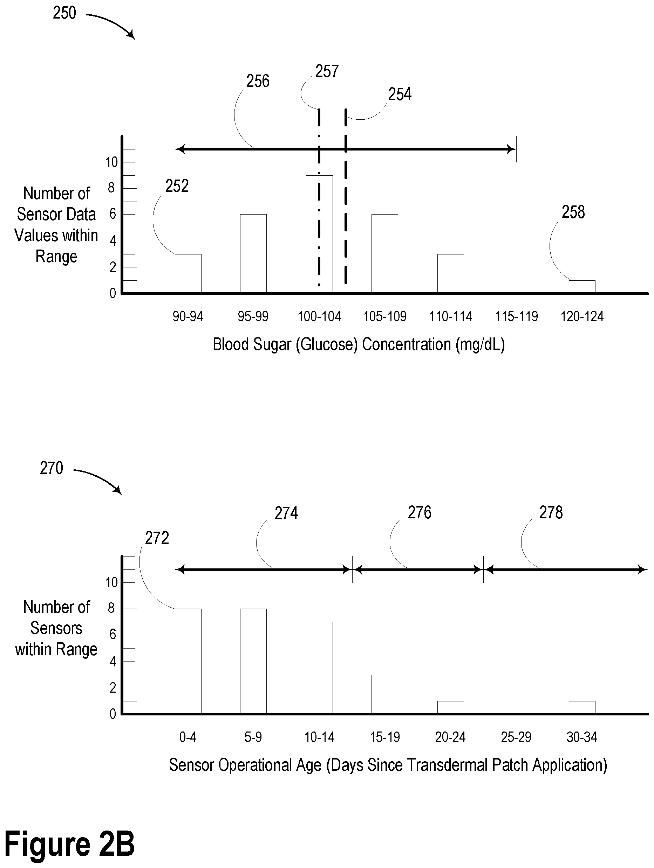

Figure 2B illustrates graphs of sensor data 250 and 260 according to example embodiments. Sensor data 250 illustrates a histogram of the number of received sensor data values that are within the blood sugar concentration ranges along the x-axis. In an example embodiment, a controller may be communicatively coupled to 28 glucose sensors applied on a living body. In such a scenario, data point 252 may represent receiving sensor data indicative of a blood sugar (glucose) concentration between 90 to 94 mg/dL from three sensors of the 28 total glucose sensors. The sensor data 250 may further include six sensors providing data indicative of a blood sugar concentration between 95-99 mg/dL, nine sensors providing data indicative of a blood sugar concentration between 100-104 mg/dL, six sensors providing data indicative of a blood sugar concentration between 105-109 mg/dL, three sensors providing data indicative of a blood sugar concentration between 110-114 mg/dL. The sensor data 250 may yet further include a single sensor providing data indicative of a blood sugar concentration between 120-124 mg/dL.

The range of data values in the sensor data 250 may vary based at least on sensor location, sensor operational age, sensor temperature, sensor battery state, sensor error, sensor-specific offset, environmental conditions, etc. It will be understood that the sensor data 250 is hypothetical and not necessarily representative of the actual range of data values that may be received by the controller. The range of data values may be greater or smaller than that illustrated by sensor data 250. Furthermore, the respective data values may be greater or smaller than those illustrated by sensor data 250. Yet further, while the illustrated scenario describes a blood sugar concentration, it should be understood that receiving a plurality of data values indicative of another physiological parameter may also include a range of data values. In some embodiments, the range of data values may tend to approximate a normal (or Gaussian) distribution, a Poisson distribution, a log-normal distribution, or another type of statistical distribution. However, in other embodiments, the range of data values need not follow a particular distribution.

As described herein, the correlated data may be determined based on a statistical analysis of the plurality of data values. For example, upon receiving sensor data from the plurality of sensors, the controller may determine an average or arithmetic mean of the values of the sensor data received. That is, the controller may add the values and then divide by the total number of values received to determine a global arithmetic mean 254. With respect to sensor data 250, the global arithmetic mean 254 may be determined to be approximately 102.7 mg/dL.

The controller may determine values that fall within a predetermined number of standard deviations based on the global arithmetic mean 254. As such, the controller may determine a standard deviation for the distribution of sensor data values and then determine which sensor data values fall within the predetermined number of standard deviations.

For example, the predetermined number of standard deviations may be two. In sensor data 250, the standard deviation may be determined to be approximately ±6.9 mg/dL. That is, approximately 68% of the sensor data values of sensor data 250 may fall within the range of 95.8 mg/dL to 109.6 mg/dL (one standard deviation from the global arithmetic mean 254). Furthermore, approximately 95% of the sensor data values of sensor data 250 may be within two standard deviations (±13.8 mg/dL) of the global arithmetic mean 254. In such a scenario, the controller may determine that sensor data values between 88.9 and 116.5 mg/dL are within two standard deviations of the global arithmetic mean 254.

The controller may discount or dismiss data values that fall outside the predetermined number of standard deviations. In the above scenario, the controller may dismiss data values of sensor data 250 indicating blood sugar concentrations outside the range from 88.9 to 116.5 mg/dL. That is, the controller may dismiss data point 258 at least because it indicates a blood sugar concentration greater than 116.5 mg/dL. In some embodiments, the controller may discount outlying data points (e.g. data point 258) by reducing a weight or by otherwise adjusting how the outlying data point is handled or considered in subsequent statistical analysis. In some examples, such dismissal or discounting of the outlying data points may reduce spurious or inaccurate data points due to, for instance, a malfunctioning sensor.

After dismissing and/or discounting outlying data points, the controller may determine a local arithmetic mean 257 based on the remaining set of data points falling within the predetermined number of standard deviations. For example, after dismissing data point 258, the controller may determine the local arithmetic mean 257 to be 102 mg/dL.

In some embodiments, the controller may determine the local arithmetic mean 257 based on a weighted average of the entire set of data points, but with outlying data points (e.g. data point 258) receiving less weight. Other ways to determine the local arithmetic mean 257 are considered within the scope of this disclosure.

In some example embodiments, the local arithmetic mean 257 may represent a more accurate and/or a more reliable indication about the physiological parameter (e.g. blood sugar concentration) at least by considering a plurality of data points and/or dismissing or discounting outlying data points.

Based on the local arithmetic mean 257, the controller may determine a health state to be "normal" at least because the blood sugar concentration is within a predetermined normal range. In such a scenario, the controller may provide an indication about the health state. For example, the controller may cause a display of a mobile device to display a message, such as "Blood Sugar: Normal".

As described herein, the correlated data is determined based, on an operational age of each sensor of the plurality of sensors. For example, the sensor data 270 may illustrate hypothetical data of the number of sensors within various ranges of operational age. That is, the sensor data 270 includes a histogram of 28 sensors based on their operational age. For example, data point 272 indicates that eight sensors are between 0-4 days since transdermal patch application. The other data points may indicate eight sensors between 5-9 days old, seven sensors between 10-14 days old, three sensors between 15-19 days old, one sensor between 20-24 days old, and one sensor between 30-34 days old.

The controller may discount or dismiss data points from the plurality of sensors based on the operational age of the respective sensor. For example, the controller may fully consider sensor data from sensors within a predetermined operational age range. With respect to sensor data 270, sensor data from sensors within a "normal" operational age range 274, e.g. less than or equal to 14 days of operational age, may be fully considered (undiscounted) in a statistical analysis, such as those described elsewhere herein.

Sensor data received from sensors within an "aging" operational age range 276, e.g. between 15-24 days of operational age, may be discounted in the statistical analysis. For example, the sensor data received from sensors in the aging operational age range 276 may receive reduced weight so as to be considered to a lesser degree compared to the sensor data from sensors in the normal operational age range 274.

Furthermore, sensor data from sensors in an "end-of-life" operational age range 278 may be discounted further or dismissed altogether. In other words, sensor data from sensors beyond the expected operational age may be given even less weight or completely discarded with respect to a subsequent statistical analysis.

In another embodiment, the operational age ranges may be determined and/or adjusted based on the reliability of data being provided by sensors within a given operational age range. That is, while sensor data may tend to become less reliable over time, the controller may periodically or continuously adjust the operational age ranges if the sensor data appears to remain reliable, even if the respective sensor is beyond a given "normal" operational age.

The one or more operational age ranges states may be predetermined or determined based on average battery life expectancy, mean time to sensor failure, environmental conditions, sensor placement location, average wear and tear conditions, sensor type, or other factors. For instance, a glucose sensor applied to a hand location may be more likely to fail before a glucose sensor applied to a back location due to factors such as wear and tear, body movement, exposure to the environment, etc.

In another embodiment, sensor data may be discounted or discarded based on another type of operational state of the respective sensor. For instance, if the respective sensor is malfunctioning (e.g. due to biofouling, temperature offset, environmental factors, or any other known or unknown reason), the corresponding data received from the malfunctioning sensor may be discounted or discarded altogether.

It will be understood that Figures 2A and 2B describe and illustrate examples of statistical analysis methods that may improve the reliability, redundancy, and/or accuracy of sensor data indicative of a physiological parameter.

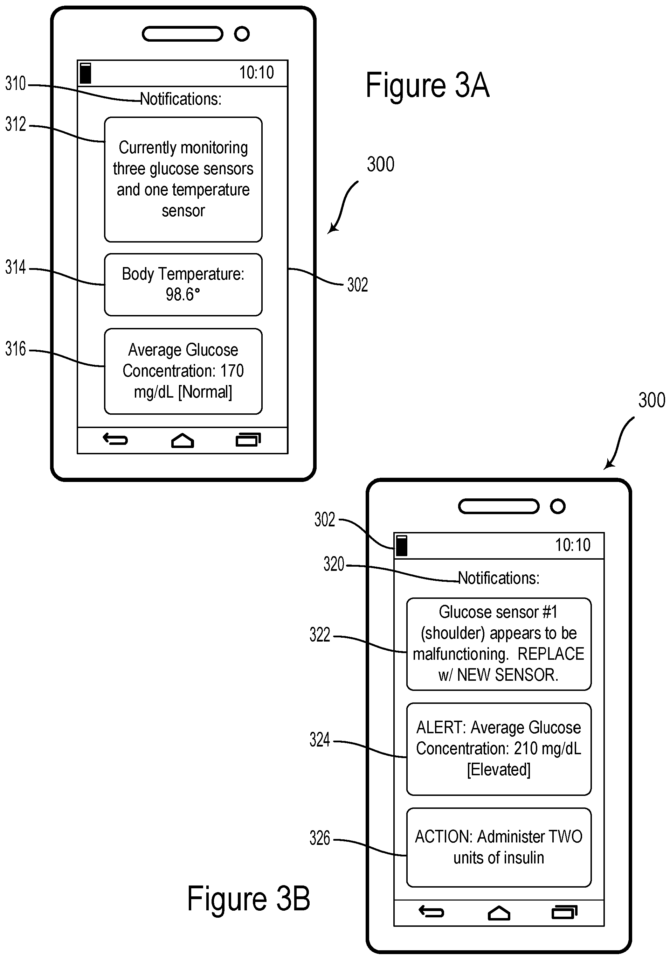

Figures 3A and 3B illustrate a mobile device 300 according to example embodiments. The mobile device 300 may be similar or identical to mobile device 110 as illustrated and described with respect to Figure 1. The mobile device 300 may include, without limitation, a smartphone, a wearable computer, a body-mountable device, or another type of computing device. The mobile device 300 may include a display 302.

Figure 3A illustrates example notifications 310 that may be displayed via display 302. For example, notifications 310 may include a sensor monitoring status message 312. The sensor monitoring status message 312 may include a number and type of sensors currently communicatively coupled to the mobile device 300. As an example, the sensor monitoring status message 312 may state "Currently monitoring three glucose sensors and one temperature sensor."

The notifications 310 may include messages related to sensor data. The messages may vary based at least on sensor type and/or the physiological parameter being measured. For example, a body temperature message 314 may state "Body Temperature: 98.6°". In such a scenario, the body temperature message 314 may be based on sensor data from an implanted temperature sensor and/or a temperature sensor otherwise proximate to the living body.

Additionally or alternatively, an average glucose concentration message 316 may state "Average Glucose Concentration: 170 mg/dL [Normal]". In such a scenario, the average glucose concentration value may be similar or identical to the local arithmetic mean of the data points related to glucose concentration, as described elsewhere herein. Other types of messages related to sensor data are possible.

Figure 3B illustrates further notifications 320 that may be provided by the mobile device 300. For example, the mobile device 300 and/or the controller may determine a malfunctioning sensor and request a user to replace it with a new sensor. In such a scenario, a sensor replacement message 322 may state "Glucose sensor #1 (shoulder) appears to be malfunctioning. REPLACE w/ NEW SENSOR."

An indication, and optionally a message, is provided in response to the controller determining a given health state. For example, in response to determining a hyperglycemic health state, the mobile device 300 may cause the display 302 to provide an alert message 324 that states "ALERT: Average Glucose Concentration: 210 ml/dL [Elevated]". Furthermore, the mobile device may provide an action message 326 that states "ACTION: Administer TWO units of insulin". It should be understood that a variety of indications and messages may be provided to a user related to one or more physiological parameters. In some embodiments, the indication may include a flashing or steady light, haptic feedback, a sound, or another type of indication to the user.

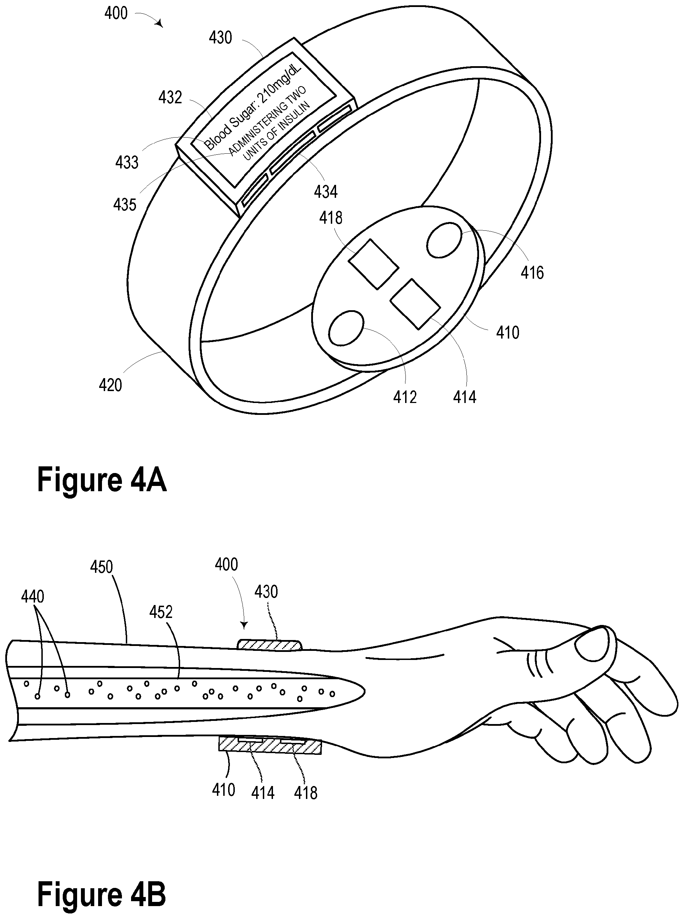

Figures 4A and 4B illustrate oblique and cross-sectional views of a body-mountable device 400 according to example embodiments. The body-mountable device 400 may be similar or identical to mobile devices 110 and 300 as illustrated and described in relation to Figures 1, 3A and 3B. As such, the body-mountable device 400 may be configured to obtain measurements related to one or more physiological parameters of a living body via a plurality of sensors. The body-mountable device 400 may be configured to be mounted to an external body surface 450 of a wearer and to enable a variety of applications and functions. The body-mountable device 400 may include a housing 430 (e.g. a rigid or semi-rigid enclosure) and a mount 420 (e.g. a strap, band, or adhesive) configured to mount a contact surface 410 of the housing 430 to the external body surface 450 of the wearer.

The body-mountable device 400 may include a plurality of sensors disposed on a portion of the contact surface 410. For instance, the sensors may include a first sensor 414 and a second sensor 418. While the contact surface 410 is mounted to the external body surface 450, the sensors may be configured to detect one or more physiological parameters related to the body of the wearer.

The first sensor 414 and/or the second sensor 418 may be configured to sense a concentration of an analyte 440 within a lumen or vasculature 452 of the wearer. In an example embodiment, the first sensor 414 and the second sensor 418 may be glucose sensors configured to measure a concentration of glucose within the body of the wearer. However, other analytes and other types of sensors are contemplated as described elsewhere herein.

The body-mountable device 400 may also include one or more drug-delivery devices 412 and 416. The drug-delivery devices 412 and 416 may be configured to deliver one or more substances to the body of the wearer of the body-mountable device. For example, the drug-delivery devices 412 and/or 416 may be configured to deliver a predetermined dose of insulin to the body of the wearer. Alternatively, the drug-delivery devices may be configured to administer other types of drugs and/or substances to the body of the wearer.

The body-mountable device 400 may include a display 432 and/or interaction devices 434 (e.g. one or more buttons, dials, touch screens, switches, etc.). In an example embodiment, the body-mountable device 400 may determine a hyperglycemic health state of a wearer. In response, the body-mountable device 400 may cause the display 432 to provide various indications and/or messages to the wearer. For example, in response to the determined hyperglycemic health state, an alert message 433 may include "Blood Sugar: 210 mg/dL."

Furthermore, in response to determining a given health state of a wearer, the body-mountable device 400 may cause an action so as to alleviate, mitigate, or otherwise manage the given health state. For example, in response to determining the hyperglycemic health state of the wearer, the body-mountable device 400 may be operable to automatically provide insulin to the wearer via the drug-delivery devices 412 and/or 416.

In such a scenario, the display 432 may provide an action indication 435. The action indication 435 may provide a notification that a drug, e.g. insulin, or another substance is being administered to the wearer. For example, the action indication 435 may state "ADMINSTERING TWO UNITS OF INSULIN". Other types of action indications are possible.

It should be understood that the body-mountable device 400 or any other similar device or system described herein may be operable to carry out a wide variety of other actions in response to determining a given health state. For example, the body-mountable device 400 may be operable to automatically contact emergency personnel in response to determining a life-threatening health state.

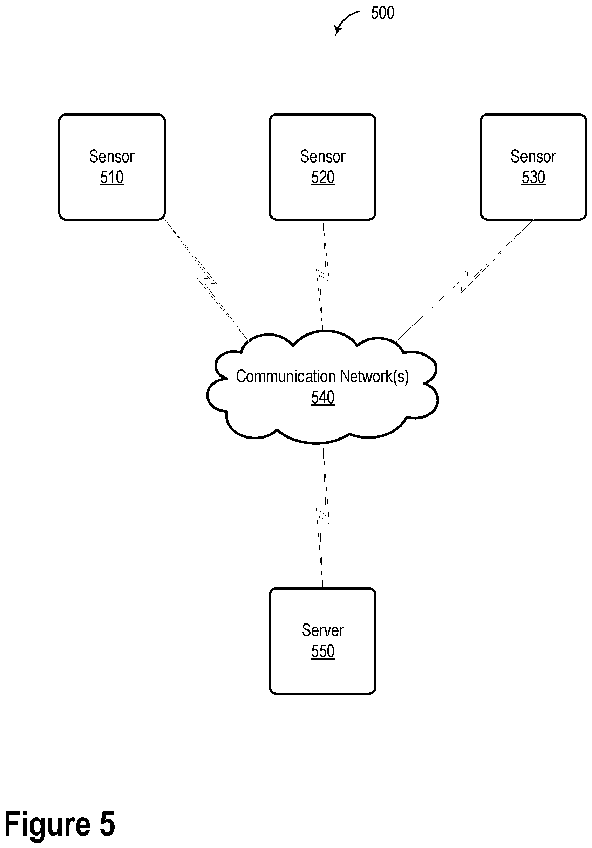

Figure 5 illustrates a system 500 according to an example embodiment. The system 500 may include a plurality of sensors, such as sensors 510, 520, and 530. The plurality of sensors may be communicatively coupled to a server 550 via one or more communication networks 540. The server 550 may represent a cloud computing platform, a computing network, or another type of computing device.

In some embodiments, sensors 510, 520, and 530 may represent the devices that aggregate sensor data from a respective plurality of sensors so as to determine a local and/or global arithmetic mean of a physiological parameter. Other network configurations are possible.

The plurality of sensors may be configured to provide sensor data indicative of one or more physiological parameters, e.g. glucose concentration, body temperature, etc., to the server 550. In such a scenario, the server 550 may be operable to receive a large amount of sensor data from the plurality of sensors. In some examples, the sensor data may be collected on a per-user account basis and the user account information may be anonymized for privacy concerns.

The server 550 may be configured to provide information, e.g. statistics, about health states within a population of anonymized user accounts. Such information may be useful to predict public health conditions and/or health trends. For example, the server 550 may be configured to predict a public health condition, such as an outbreak of influenza, increasing rate of diabetes, or heart disease. Other types of public health conditions may be determined by the server 550.

### Unclaimed Method Examples

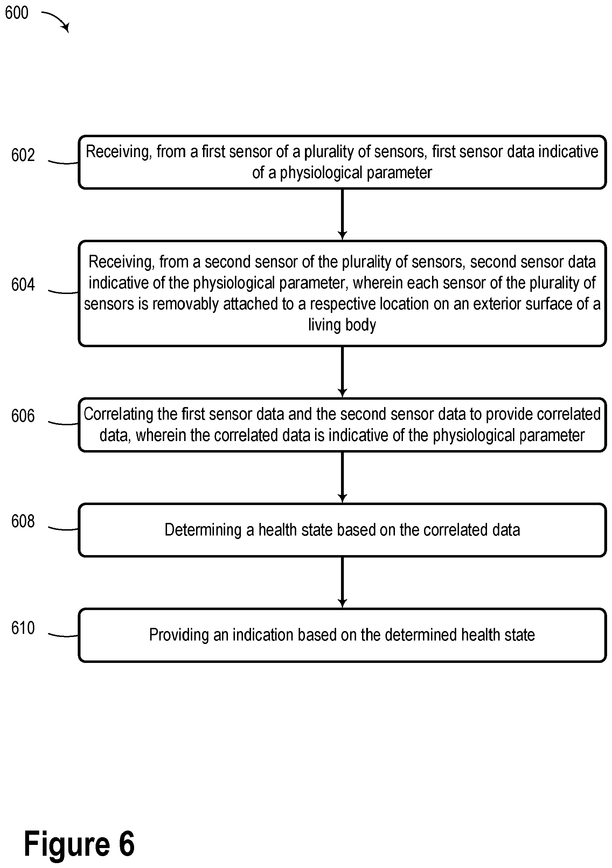

Figure 6 illustrates a method 600, according to an unclaimed example. The method 600 includes blocks that may be carried out in any order. The method 600 may correspond to blocks or steps that may be carried out using any or all of the devices and/or systems illustrated and described in reference to Figures 1, 2, 3A, 3B, 4A, 4B, and 5.

Block 602 includes receiving, from a first sensor of a plurality of sensors, first data indicative of a physiological parameter. The first sensor may be identical or similar to first sensor 132 as illustrated and described in relation to Figure 1. The first data may include signals received via a wireless communication link. For example, the first data may be received via BLUETOOTH Low Energy (BLE) communication link or a near-field communication (NFC) link. The physiological parameter may include a concentration of an analyte, a body temperature, a galvanic skin response, a blood pressure, a pulse rate, etc. The analyte may include one or more analytes of interest so as to determine a health state condition. For example, the analyte may include glucose in blood, white blood cells, red blood cells, a biomarker, a fluorescent tag, or another indicator of a particular health state.

Examples may include a mobile device (e.g. mobile device 110 as illustrated and described with respect to Figure 1) or another type of computing device receiving the first data from the first sensor. The first data may include information indicative of a glucose concentration in a living body.

Block 604 includes receiving, from a second sensor of the plurality of sensors, second sensor data indicative of the physiological parameter. The second sensor may be similar or identical to second sensor 134 as illustrated and described with respect to Figure 1. In other words, a mobile device, such as mobile device 110, may be operable to receive second sensor data from the second sensor. The second sensor data may include data indicative of the physiological parameter, such as a blood glucose concentration or a body temperature.

Each sensor of the plurality of sensors is removably attached to a respective location on an exterior surface of a living body. That is, the sensors may be associated with, or incorporated into a removable transdermal patch. In an example embodiment, the transdermal patch may include one or more sensors. The one or more sensors may interact with the body (e.g. sense one or more physiological parameters) via one or more microneedles. In some embodiments, the first sensor, the second sensor, or another sensor of the plurality of sensors may be configured to be implanted into the living body. For example, implantable temperature sensors, implantable glucose sensors, and/or other types of *in vivo* sensors configured to sense a physiological parameter are contemplated herein.

Block 606 includes correlating the first sensor data and the second sensor data to provide correlated data. The correlated data is indicative of the physiological parameter. For instance, the first sensor data and the second sensor data may both relate to the physiological parameter, such as a blood sugar (glucose) concentration. In such a scenario, the first sensor data and the second sensor data may provide information indicative of similar, but slightly different glucose concentrations from the same living body.

Correlating the first sensor data and the second sensor data may include averaging the first and second sensor data. For instance, a mobile device may receive sensor data from a plurality of sensors (e.g. 2, 10, 100, or more sensors) that could include a statistical range of values. As such, correlating the sensor data may include taking a statistical average (e.g. an arithmetic mean) of the plurality of sensor data values.

In some examples, correlating the sensor data may include weighting sensor data values based on a confidence level associated with one or more sensors. That is, a sensor may provide a data value more than a threshold number of standard deviations away from the statistical average of the plurality of data values. In such a scenario, the data value from the "outlier" sensor may be discounted, discarded, or associated with a smaller weight compared to those data with values within the threshold number of standard deviations from the statistical average of data values.

Block 608 includes determining a health state based on the correlated data. That is, the correlated data may indicate that a physiological parameter is in a typical/normal/safe range or an atypical/abnormal/unsafe range. For example, the correlated data from a plurality of glucose sensors may indicate that an average concentration of blood sugar is 210 mg/dL. In such a scenario, the controller and/or the mobile device may determine a hyperglycemic health state. A variety of health states are contemplated herein, including, but not limited to: a normal/healthy health state, hypoglycemia, fever, drug overdose, poisoning, elevated stress, high blood pressure, hypertension, arterial blockage, myocardial infarction (heart attack), infection, lack of sleep, vitamin deficiency, etc.

Block 610 includes providing an indication based on the determined health state. The indication may include information about the physiological parameter and/or actions that should be taken in an effort to alleviate or otherwise change the determined health state. For example, in response to determining a hyperglycemic health state, the mobile device and/or the controller may cause a display of the mobile device to provide an indication such as, "Average Blood Sugar = 210 mg/dL, Please administer two units of insulin." It will be understood that the content of the indication may depend at least in part on the determined health state. Thus, the indication may include a variety of information and/or actions that should be taken. In another example embodiment, in response to the determined health state being an arterial blockage, the indication may include, "Arterial blockage detected, seek medical attention immediately!"

In some cases, the mobile device and/or the controller may take additional or alternative action in response to particular determined health states. For example, in response to a hyperglycemic health state, the mobile device may cause a drug administration device to administer a determined amount of insulin. The drug administration device may include an insulin pump and/or one or more microneedles configured to deliver insulin. In the arterial blockage scenario, the mobile device and/or the controller may be configured to alert emergency personnel to the user's physiological condition and position.

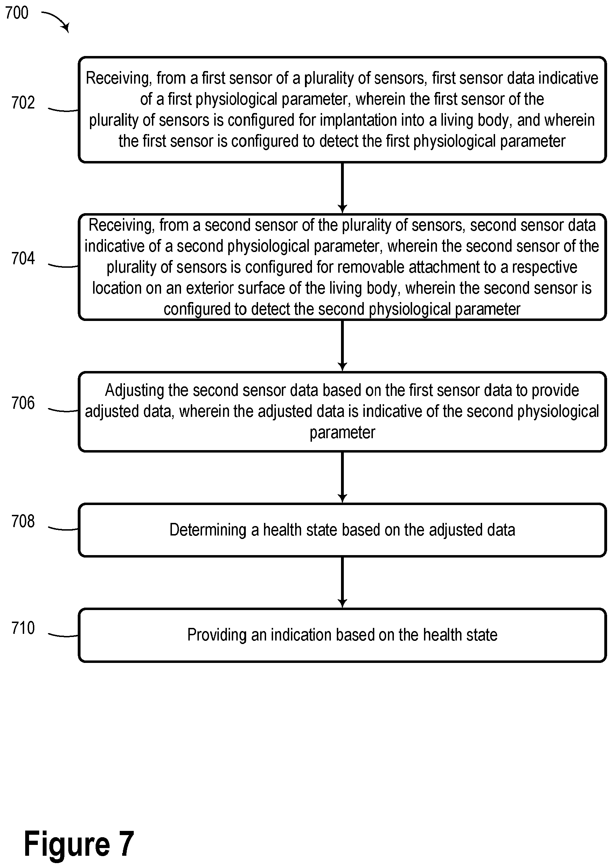

Figure 7 illustrates a method 700, according to an unclaimed example. The method 700 includes blocks that may be carried out in any order. The method 700 may correspond to blocks or steps that may be carried out using any or all of the devices and/or systems illustrated and described in reference to Figures 1, 2, 3A, 3B, 4A, 4B, and 5.

Block 702 includes receiving, from a first sensor of a plurality of sensors, first sensor data indicative of a first physiological parameter. The first sensor is configured for implantation into a living body. In an example embodiment, the first sensor may include an implantable temperature sensor, however other types of sensors are contemplated. The first sensor is configured to detect the first physiological parameter. The first physiological parameter may include a body temperature or another measurable characteristic of the living body.

Block 704 includes receiving, from a second sensor of the plurality of sensors, second sensor data indicative of a second physiological parameter. The second sensor is configured for removable attachment to a respective location on an exterior surface of the living body. As such, the second sensor may be a transdermal sensor or another type of removable sensor that may be coupled to an outer surface of the body. The second sensor is configured to detect the second physiological parameter, which may be a concentration of glucose, a concentration of glycated hemoglobin, a pulse rate, a blood pressure, or another measurable physiological parameter described herein.

Block 706 includes adjusting the second sensor data based on the first sensor data to provide adjusted data. That is, the second sensor may have a temperature-dependent offset and/or temperature-dependent device performance. In such a scenario, temperature (or other relevant data) from the first sensor may be used to adjust, calibrate, or otherwise modify the second sensor data to form the adjusted data. In some examples, the adjusted data may represent a more accurate measurement of the physiological parameter (e.g. glucose concentration).

Block 708 includes determining a health state based on the adjusted data. As described elsewhere herein, a health state may be determined from information indicative of a physiological parameter. In the present scenario, the adjusted data may include information from the second sensor calibrated with respect to information obtained from the first sensor. Based on the adjusted data, a health state, such as a hypo- or hyperglycemic state may be determined. Other types of health states are possible and contemplated herein.

Block 710 includes providing an indication based on the health state. In some example embodiments, the indication may include displaying an alert notification or an action notification via a display of a mobile device. For instance, if the determined health state is a hyperglycemic state, the indication may include an alert notification via a smartphone or tablet that states: "Elevated glucose concentration, please administer insulin."

## Claims

1. A system (100) comprising:
a plurality of sensors (132, 134, 138) for measuring a physiological parameter; and
a controller (110) comprising a memory (124) and a processor (122), wherein the memory stores instructions that are executable by the processor to cause the controller to perform operations comprising:
receiving, from a first sensor (132) of the plurality of sensors, first sensor data indicative of the physiological parameter;
receiving, from a second sensor (134) of the plurality of sensors, second sensor data indicative of the physiological parameter;
correlating the first sensor data and the second sensor data to provide correlated data, wherein the correlated data is indicative of the physiological parameter;
determining a health state based on the correlated data; and
providing an indication based on the determined health state;
**characterized in that**:
at least one sensor of the plurality of sensors is configured for removable attachment to a respective location on an exterior surface of a living body; and
correlating the first sensor data and the second sensor data includes weighting each of the first sensor data and the second sensor data based on an operational age of the respective sensor.

2. The system (100) of claim 1, wherein at least one sensor of the plurality of sensors is configured for removable attachment to the living body via a transdermal patch.

3. The system (100) of claim 2, wherein the transdermal patch comprises at least two sensors of the plurality of sensors.

4. The system (100) of claim 1, wherein the physiological parameter comprises a concentration of an analyte.

5. The system (100) of claim 4, wherein the analyte comprises glucose.

6. The system (100) of claim 5, wherein the health state is a hyperglycemic state or a hypoglycemic state, and wherein the indication comprises a high glucose alert or a low glucose alert.

7. The system (100) of claim 1, wherein the plurality of sensors are configured to communicate with the controller via at least one of: a near field communication (NFC) link, a BLUETOOTH Low Energy (BLE) link, an ultra high frequency (UHF) radio frequency identification (RFID) link, or a WiFi link.

8. The system (100) of claim 1, wherein correlating the first sensor data and the second sensor data to provide correlated data comprises discounting the first sensor data based on an operational state of the first sensor.

## Patentansprüche

1. System (100), umfassend:
eine Vielzahl von Sensoren (132, 134, 138) zum Messen eines physiologischen Parameters; und
eine Steuerung (110), umfassend einen Speicher (124) und einen Prozessor (122), wobei der Speicher Befehle speichert, die durch den Prozessor ausführbar sind, um zu bewirken, dass die Steuerung Vorgänge durchführt, die Folgende umfassen:
Empfangen von ersten Sensordaten, die den physiologischen Parameter angeben, von einem ersten Sensor (132) aus der Vielzahl von Sensoren;
Empfangen von zweiten Sensordaten, die den physiologischen Parameter angeben, von einem zweiten Sensor (134) aus der Vielzahl von Sensoren;
Korrelieren der ersten Sensordaten und der zweiten Sensordaten, um korrelierte Daten bereitzustellen, wobei die korrelierten Daten den physiologischen Parameter angeben;
Bestimmen eines Gesundheitszustands basierend auf den korrelierten Daten; und
Bereitstellen einer Angabe basierend auf dem bestimmten Gesundheitszustand;
**dadurch gekennzeichnet, dass**:
zumindest ein Sensor aus der Vielzahl von Sensoren für eine lösbare Anbringung an einer entsprechenden Stelle auf einer Außenoberfläche eines lebenden Körpers ausgelegt ist; und
das Korrelieren der ersten Sensordaten und der zweiten Sensordaten das Gewichten jeder aus den ersten Sensordaten und den zweiten Sensordaten basierend auf einem Betriebsalter des entsprechenden Sensors umfasst.

2. System (100) nach Anspruch 1, wobei zumindest ein Sensor aus der Vielzahl von Sensoren zur lösbaren Anbringung an dem lebenden Körper über ein transdermales Pflaster ausgelegt ist.

3. System (100) nach Anspruch 2, wobei das transdermale Pflaster zumindest zwei Sensoren aus der Vielzahl von Sensoren umfasst.

4. System (100) nach Anspruch 1, wobei der physiologische Parameter eine Konzentration eines Analyten umfasst.

5. System (100) nach Anspruch 4, wobei der Analyt Glucose umfasst.

6. System (100) nach Anspruch 5, wobei der Gesundheitszustand ein hyperglykämischer Zustand oder ein hypoglykämischer Zustand ist und wobei die Angabe einen Hochglucose-Alarm oder einen Niederglucose-Alarm umfasst.

7. System (100) nach Anspruch 1, wobei die Vielzahl von Sensoren ausgelegt ist, um über zumindest eines der Folgenden mit der Steuerung zu kommunizieren: Nahfeldkommunikations- (NFC-) Verbindung, einer BLUETOOTH-Niederenergie- (BLE-) Verbindung, einer Ultrahochfrequenz- (UHF-) Funkfrequenzidentifikations- (RFID-) Verbindung oder einer WLAN-Verbindung.

8. System (100) nach Anspruch 1, wobei das Korrelieren der ersten Sensordaten und der zweiten Sensordaten zum Bereitstellen von korrelierten Sensordaten das Nicht-Berücksichtigen der ersten Sensordaten basierend auf einem Betriebszustand des ersten Sensors umfasst.

## Revendications

1. Système (100), comprenant :
une pluralité de capteurs (132, 134, 138) pour mesurer un paramètre physiologique ; et
un dispositif de commande (110) comprenant une mémoire (124) et un processeur (122), dans lequel la mémoire stocke des instructions qui sont exécutables par le processeur pour amener le dispositif de commande à effectuer des opérations comprenant :
recevoir, à partir d'un premier capteur (132) de la pluralité de capteurs, des premières données de capteur indicatives du paramètre physiologique ;
recevoir, à partir d'un second capteur (134) de la pluralité de capteurs, des secondes données de capteur indicatives du paramètre physiologique ;
mettre en corrélation les premières données de capteur et les secondes données de capteur pour fournir des données corrélées, dans lequel les données corrélées sont indicatives du paramètre physiologique ;
déterminer un état de santé sur la base des données corrélées ; et fournir une indication sur la base de l'état de santé déterminé ;
**caractérisé en ce que** :
au moins un capteur de la pluralité de capteurs est configuré pour une fixation amovible à un emplacement respectif sur une surface extérieure d'un corps vivant ; et
la mise en corrélation des premières données de capteur et des secondes données de capteur comprend une pondération de chacune des premières données de capteur et des secondes données de capteur sur la base d'un âge de fonctionnement du capteur respectif.

2. Système (100) selon la revendication 1, dans lequel au moins un capteur de la pluralité de capteurs est configuré pour une fixation amovible au corps vivant via un timbre transdermique.

3. Système (100) selon la revendication 2, dans lequel le timbre transdermique comprend au moins deux capteurs de la pluralité de capteurs.

4. Système (100) selon la revendication 1, dans lequel le paramètre physiologique comprend une concentration d'un analyte.

5. Système (100) selon la revendication 4, dans lequel l'analyte comprend du glucose.

6. Système (100) selon la revendication 5, dans lequel l'état de santé est un état hyperglycémique ou un état hypoglycémique, et dans lequel l'indication comprend une alerte de glycémie élevée ou une alerte de glycémie faible.

7. Système (100) selon la revendication 1, dans lequel la pluralité de capteurs sont configurés pour communiquer avec le dispositif de commande via au moins une parmi : une liaison de communication en champ proche (NFC), une liaison BLUETOOTH basse énergie (BLE), une liaison d'identification par radiofréquence (RFID) à ultra haute fréquence (UHF), ou une liaison WiFi.

8. Système (100) selon la revendication 1, dans lequel la mise en corrélation des premières données de capteur et des secondes données de capteur pour fournir des données corrélées comprend une actualisation des premières données de capteur sur la base d'un état opérationnel du premier capteur.
